# EUROPEAN PATENT APPLICATION

(11) **EP 4 710 915 A1**
(43) Date of publication of application: **18.03.2026**
(21) Application number: 24803416.7
(22) Date of filing: 26.04.2024
(51) Int. Cl.: A61K 8/34, A61K 31/045, A61P 17/16, A61P 29/00, A61Q 19/00

(54) **HUMECTANT, SKIN BARRIER FUNCTION IMPROVING AGENT, ANTI-INFLAMMATORY AGENT, HYALURONIC ACID PROMOTING AGENT, AND MELANIN PRODUCTION INHIBITOR**

(30) Priority: 10.05.2023 JP 2023078123
(71) Applicant: Shiseido Company, Ltd., Chuo-ku Tokyo 104-0061 (JP)
(72) Inventor: NISHIZUKA, Taichi, Tokyo 104-0061 (JP); NAKANISHI, Shinobu, Tokyo 104-0061 (JP); YANG, Shengfan, Tokyo 104-0061 (JP)
(74) Representative: Ter Meer Steinmeister & Partner
(86) International application number: PCT/JP2024/016558
(87) International publication number: WO 2024/232303

(57) **Abstract**

Provided are a moisturizer, a skin barrier function improving agent, an anti-inflammatory agent, a hyaluronic acid increasing agent, and a melanin production inhibitor.

A moisturizer, a skin barrier function improving agent, an anti-inflammatory agent, a hyaluronic acid increasing agent, and a melanin production inhibitor, containing cis-3-hexenol as an active component, are provided.

## Description

### FIELD

The present invention relates to a moisturizer, a skin barrier function improving agent, an anti-inflammatory agent, a hyaluronic acid increasing agent, and a melanin production inhibitor, containing cis-3-hexenol as an active component.

### BACKGROUND

Cis-3-hexenol is an aroma component found in green tea, and smelling the scent thereof is known to have a fatigue-relieving effect (PTL 1). However, the effect is exhibited by smelling the aroma and differs from the effect when cis-3-hexenol is applied directly to the skin. PTL 2 describes that cis-3-hexenol has an effect of inhibiting melanin production by melanoma cells.

### [CITATION LIST]

### [PATENT LITERATURE]

[PTL 1] WO 2005/000286
[PTL 2] Japanese Unexamined Patent Publication (Kokai) No. 2022-174825

### SUMMARY

### [TECHNICAL PROBLEM]

The present inventors aim to obtain a moisturizer, a skin barrier function improving agent, an anti-inflammatory agent, a hyaluronic acid increasing agent, and a melanin production inhibitor.

### [SOLUTION TO PROBLEM]

The present inventors have discovered that applying cis-3-hexenol directly to the skin brings about preferable effects on the skin, such as moisturization, skin barrier function improvement, anti-inflammation, hyaluronic acid increase, and melanin production inhibition, and thus arrived at the present invention.

The present invention relates to the following:
[1] A moisturizer containing cis-3-hexenol as an active component.
[2] A skin barrier function improving agent containing cis-3-hexenol as an active component.
[3] An anti-inflammatory agent containing cis-3-hexenol as an active component.
[4] A hyaluronic acid increasing agent containing cis-3-hexenol as an active component.
[5] A melanin production inhibitor containing cis-3-hexenol as an active component.
[6] An FLG gene expression promoter containing cis-3-hexenol as an active component.
[7] A GBA gene expression promoter containing cis-3-hexenol as an active component.
[8] A COX2 gene expression inhibitor containing cis-3-hexenol as an active component.
[9] An HAS2 gene expression promoter containing cis-3-hexenol as an active component.
[10] An MIF gene expression inhibitor containing cis-3-hexenol as an active component.

### [ADVANTAGEOUS EFFECTS OF INVENTION]

By directly applying cis-3-hexenol, effects of moisturization, barrier function improvement, anti-inflammation, hyaluronic acid increase, and melanin production inhibition of the skin can be expected.

### DESCRIPTION OF EMBODIMENTS

Cis-3-hexenol (CAS number: 928-96-1) is an unsaturated alcohol that, along with the isomer trans-2-hexenol, is also known as leaf alcohol, and is an aroma component contained in green tea, etc.

As stated above, PTL 1 describes that the aroma of cis-3-hexenol exerts an anti-fatigue effect. However, the effect is exhibited by smelling the aroma. In the Examples, the effect is actually demonstrated by having test subjects inhaling cis-3-hexenol. PTL 2 describes that cis-3-hexenol has an effect of inhibiting melanin production by melanoma cells. The present inventors have discovered that applying cis-3-hexenol directly to the skin brings about preferable effects on the skin, such as moisturization, skin barrier function improvement, anti-inflammation, hyaluronic acid increase, and melanin production inhibition.

The present invention provides a moisturizer, a skin barrier function improving agent, an anti-inflammatory agent, a hyaluronic acid increasing agent, and a melanin production inhibitor (hereinafter, sometimes collectively referred to as agent of the present invention), containing cis-3-hexenol as an active component.

Moisturization, skin barrier function improvement, anti-inflammation, hyaluronic acid increase, and melanin production inhibition, for example, can be confirmed by measuring an increase in gene expression levels and protein production of FLG, GBA, and HAS2 and a decrease in gene expression levels and protein production of COX2 and MIF in biological samples such as keratinocytes and skin fibroblasts. Measurement of gene expression levels can be carried out by known methods in the present technical field, such as quantitative PCR and northern blotting. For example, probes targeting mRNA of FLG, GBA, COX2, HAS2, and/or MIF may be used. Protein levels can be measured using known methods in the present technical field, such as western blotting, immunostaining, ICM, and ELISA. In addition to the methods stated above, moisturization, skin barrier function improvement, anti-inflammation, hyaluronic acid increase, and melanin production inhibition, for example, can be confirmed by any method such as visual inspection, TEWL, stratum corneum thickness, measurement of specific proteins or lipids, or measurement of hyaluronic acid levels by ELISA in skin samples such as skin models and skin cells such as fibroblasts.

The present invention also provides an FLG gene expression promoter, a GBA gene expression promoter, a COX2 gene expression inhibitor, an HAS2 gene expression promoter, and an MIF gene expression inhibitor, containing cis-3-hexenol as an active component. In one aspect, the FLG gene expression promoter promotes FLG gene expression in epidermal keratinocytes. In one aspect, the GBA gene expression promoter promotes GBA gene expression in epidermal keratinocytes. In one aspect, the COX2 gene expression inhibitor inhibits COX2 gene expression in epidermal keratinocytes. In one aspect, the HAS2 gene expression promoter promotes HAS2 gene expression in skin fibroblasts. In one aspect, the MIF gene expression inhibitor inhibits MIF gene expression in skin keratinocytes.

FLG (Filaggrin) is a gene encoding filaggrin, which is involved in the moisture retention function of the skin as an NMF. GBA (β-Glucocerebrosidase) is a gene encoding an enzyme that produces ceramide EOP (ceramide 1), which is related to the barrier function of the skin. COX2 (Prostaglandin-endoperoxide synthase 2) is a gene encoding an enzyme that produces PGE2, which is involved as an inflammatory mediator and in melanin production. HAS2 (Hyaluronan synthase 2) is a gene encoding an enzyme that synthesizes hyaluronic acid, which is produced in dermal cells and is involved in moisture retention of the skin. MIF (Macrophage migration inhibitory factor) is a gene encoding an enzyme that prevents migration of macrophages, hinders decomposition of tyrosinase protein, and enhances melanin production.

Measurement of gene expression, as stated above, can be carried out by known methods in the present technical field, such as quantitative PCR and northern blotting. Promotion of gene expression, in one embodiment, means an increase that is statistically significant (for example, Student's t-test or Dunnett's test) at a significance level of 5%, and/or an increase of, for example, 10% or greater, 20% or greater, 30% or greater, 40% or greater, 50% or greater, 60% or greater, 70% or greater, 80% or greater, 90% or greater, 100% or greater, or even greater. Inhibition of gene expression, for example, means a decrease that is statistically significant (for example, Student's t-test or Dunnett's test) at a significance level of 5%, and/or a decrease of, for example, 10% or greater, 20% or greater, 30% or greater, 40% or greater, 50% or greater, 60% or greater, 70% or greater, 80% or greater, 90% or greater, or 100%.

Measurement of protein expression, as stated above, can be carried out by known methods in the present technical field, such as western blotting, immunostaining, ICM, and ELISA. Promotion of protein expression, in one embodiment, means an increase that is statistically significant (for example, Student's t-test or Dunnett's test) at a significance level of 5%, and/or an increase of, for example, 10% or greater, 20% or greater, 30% or greater, 40% or greater, 50% or greater, 60% or greater, 70% or greater, 80% or greater, 90% or greater, 100% or greater, or even greater. Inhibition of protein expression, for example, means a decrease that is statistically significant (for example, Student's t-test or Dunnett's test) at a significance level of 5%, and/or a decrease of, for example, 10% or greater, 20% or greater, 30% or greater, 40% or greater, 50% or greater, 60% or greater, 70% or greater, 80% or greater, 90% or greater, or 100%.

Measurement of hyaluronic acid can be carried out by known methods in the present technical field such as ELISA, for example, sandwich ELISA. An increase in hyaluronic acid, in one embodiment, means an increase that is statistically significant (for example, Student's t-test or Dunnett's test) at a significance level of 5%, and/or an increase of, for example, 10% or greater, 20% or greater, 30% or greater, 40% or greater, 50% or greater, 60% or greater, 70% or greater, 80% or greater, 90% or greater, 100% or greater, or even greater.

The biological sample may be a sample in which the effects of moisturization, skin barrier function improvement, anti-inflammation, hyaluronic acid increase, and melanin production inhibition can be measured, or a cell culture in which expression levels of genes such as FLG, GBA, COX2, HAS2, and/or MIF, protein production levels corresponding to these genes, and/or hyaluronic acid production levels can be measured. For example, preferably keratinocyte or fibroblast can be used. Alternatively, the biological sample may be a skin sample, or a 3D-constructed cultured skin model may be used. The cell may be derived from any animal, but from the viewpoint of development of cosmetics and pharmaceuticals, is preferably derived from a human.

The present invention also provides a method for moisturization, skin barrier function improvement, anti-inflammation, hyaluronic acid increase, and melanin production inhibition in a subject, comprising applying cis-3-hexenol, the agent of the present invention, or a composition comprising the same.

Examples of subjects to which the method of the present invention is applied include subjects who have dry skin and require or desire moisturization, subjects who have reduced skin barrier function or desire improvement thereof, subjects who have inflammation and require inhibition or desire prevention thereof, subjects who are deficient in hyaluronic acid or desire prevention thereof, and subjects who have reduced gene expression levels or protein production levels of FLG, GBA, and/or HAS2 or increased gene expression levels or protein production levels of COX2 and/or MIF.

The method according to the present application is for cosmetic purposes and may exclude medical acts carried out by a physician or medical professional. In addition, the method according to the present application may be a method for supporting a cosmetic act of a subject.

The present invention further provides a composition for moisturization, skin barrier function improvement, anti-inflammation, hyaluronic acid increase, and melanin production inhibition, comprising cis-3-hexenol or the agent of the present invention. The composition of the present invention may be a composition for moisturization, skin barrier function improvement, anti-inflammation, hyaluronic acid increase, and melanin production inhibition. The composition may be a cosmetic, a pharmaceutical, or a quasi-drug.

For the application, the composition can be applied via any route, such as transdermal, oral, transmucosal, nasal, intravenous, intraarterial, or subcutaneous, but from the viewpoint of having an effect on the skin, is preferably applied transdermally, i.e., to the skin. For the skin, the composition can be applied to any part thereof, such as the face, head, neck, limbs, or torso.

In the case of a cosmetic, the composition can be blended into a face or body cosmetic such as a toner, an emulsion, a serum, a cream, a lotion, a pack, an essence, or a gel; a makeup cosmetic such as a foundation, a cosmetic primer, or a concealer; or even a bath additive. In the case of a pharmaceutical for transdermal administration, the composition can be formulated into an external skin preparation. The form needs only to be applicable to the skin and is not particularly limited. For example, any dosage formulation can be applied, such as a solution, an emulsion, a solid, a semi-solid, a powder, a powder dispersion, a water-oil bilayer separation, a water-oil-powder trilayer separation, an ointment, a gel, an aerosol, a mousse, or a stick. In addition, bases and excipients commonly used in cosmetics and external skin preparations, such as preservatives, emulsifiers, and pH adjusters, may be used.

The amount of active component in the agent or composition of the present invention can be arbitrarily selected from the viewpoint of exhibiting an effect of moisturization, skin barrier function improvement, anti-inflammation, and/or hyaluronic acid increase. For example, cis-3-hexenol can be blended at 0.0005 to 100.0 mM. From the viewpoint of sufficiently exhibiting the effect, preferably 0.05 mM or more can be blended and, for example, 0.5 mM or more can be blended. Since cis-3-hexenol has an aroma, from the viewpoint of avoiding an excessively strong odor, preferably 10.0 mM or less can be blended, and more preferably 5.0 mM or less may be blended. The components described above may be combined in any ratio, and in that case, the total amount of the components is preferably within the above range.

The present invention further provides cis-3-hexenol for moisturization, skin barrier function improvement, anti-inflammation, and/or hyaluronic acid increase, wherein preferably, the moisturization, skin barrier function improvement, anti-inflammation, and/or hyaluronic acid increase is mediated by the promotion of FLG, GBA, and HAS2 gene expressions and/or the inhibition of COX2 gene expression. In one aspect, the moisturization consists of moisturization of the epidermis. In one embodiment of the aspect, the moisturization of the epidermis is exhibited by promoting gene expression or protein production of FLG or GBA in keratinocytes and/or gene expression or protein production of HAS2 in skin fibroblasts. In one aspect, the skin barrier function improvement consists of a skin barrier function improvement of the epidermis. In one embodiment of the aspect, the skin barrier function improvement of the epidermis is exhibited by promoting gene expression or protein production of GBA in keratinocytes. In one aspect, the anti-inflammation consists of anti-inflammation of the epidermis. In one embodiment of the aspect, the anti-inflammation of the epidermis is exhibited by inhibiting gene expression or protein production of COX2 in keratinocytes. In one aspect, the hyaluronic acid increase consists of a dermal hyaluronic acid increase. In one embodiment of the aspect, the dermal hyaluronic acid increase is exhibited by promoting gene expression or protein production of HAS2 in skin fibroblasts. In one aspect, the melanin production inhibition consists of melanin production inhibition of the epidermis, and is exhibited by inhibiting MIF and/or COX2 skin gene expression or protein production in keratinocytes.

The present invention also provides a use of cis-3-hexenol in the manufacture of drugs for moisturization, skin barrier function improvement, anti-inflammation, hyaluronic acid increase, and melanin production inhibition.

All literatures mentioned in the present specification are incorporated herein by reference in the entirety thereof.

The Examples of the present invention described below are intended for exemplification only, and do not limit the technical scope of the present invention. The technical scope of the present invention is limited only by the description of the claims. Modifications to the present invention, for example, addition, deletion, and substitution of constituent elements of the present invention, can be made without departing from the spirit of the present invention.

### EXAMPLES

To confirm the effects of moisturization, skin barrier function improvement, anti-inflammation, hyaluronic acid increase, and melanin production inhibition of cis-3-hexenol, expressions of genes involved in these functions were examined by analysis. More specifically, expression levels of FLG, GBA, COX2, HAS2, and MIF were measured according to the methods described below.

### Example 1: Effect of cis-3-hexenol on keratinocyte

### 1-1: Cell culture

HaCaT cells were seeded in Dulbecco's Modified Eagle Medium (DMEM, Cat No. 043-30085, Wako, Japan) comprising 10.0% (v/v) Fetal Bovine Serum (FBS, Cat No. SH30071.03, Hyclone, UK) and 1.0% (v/v) antifungal agent (Antibiotic-Antimycotic 100X, Cat No. 15240-062, Invitrogen, USA) in a 24-well plate (Cat No. 3526, Corning, USA) at a density of 10.0 × 10⁴ cells/well and cultured within a CO2 incubator (CO2 concentration of 5%, 37°C) for 24 h. The medium was removed and then replaced with a medium to which cis-3-hexenol (CAS: 928-96-1, Wako Pure Chemical Industries, Ltd.) was added so as to have the final concentration shown in the table below, and the cells were further cultured within the CO2 incubator for 48 h. As the control, a medium not comprising cis-3-hexenol was used.

### 1-2: RNA extraction and purification, quantification, and purity measurement

RNA extraction and purification were carried out using a PureLink^{™} RNA Mini Kit (Cat No. 12183018A, Invitrogen, USA), a portion of the purified RNA was aliquoted into a UV-transparent 96-well plate and diluted 10-fold with Tris-EDTA Buffer, and absorbances (OD230, OD260, and OD280) at 230 nm, 260 nm, and 280 nm were measured using a microplate reader (SPARK^{®}, 10M TECAN, Switzerland). RNA concentrations were calculated using OD260 and diluted with TE Buffer to adjust the RNA concentrations to 10 µg/mL.

### 1-3: Gene expression analysis by real-time PCR method

Reverse transcription of RNA was carried out using SuperScript ^{™} IV VILO^{™} Master Mix with exDNase (Cat No. 11766050, Invitrogen, USA). 4 µL of SuperScript ^{™} IV VILO^{™} Master Mix and 6 µL of Nuclease-free Water were added per well in an 8-tube strip and heated at 25°C for 10 min, 50°C for 10 min, and 85°C for 5 min using real-time PCR (QuantStudio^{®} 3, Applied Biosystems, USA) to synthesize cDNA. 10 µL of TaqMan^{®} Fast Advanced Master Mix (Cat No. 4444557, Applied Biosystems, USA), 1 µL of TaqMan Gene Expressior, 7 µL of UltraPure Distilled Water (Invitrogen, Cat No. 10977-015, USA), and 2 µL of cDNA were added per well in a PCR plate and sealed. A primer (Hs00856927_g1) for FLG, a primer (Hs00986836_g1) for GBA, a primer (Hs00153133_m1) for COX2, or a primer (Hs00236988_g1) for MIF (macrophage migration inhibitory factor) and a primer (Hs02786624_g1) for GAPDH as an internal standard gene were used to carry out real-time qPCR, the threshold cycle (Ct) value of each gene such as FLG in the cis-3-hexenol-added medium was calculated, and then Ct value was corrected with GAPDH to obtain a ΔCt value. Assuming that gene level doubles per cycle, when gene expression level of the control was set at 1, the gene expression level in the cis-3-hexenol-added medium was determined. In the analysis of gene expression level, the average value of 24-well plate × 3 wells was used for each treatment group.

Results are shown in Table 1 below.

**[Table 1]**

| Table 1: Promotion of FLG expression due to cis-3-hexenol addition | | |
|---|---|---|
| | cis-3-Hexenol unadded | cis-3-Hexenol 0.5 mM |
| Gene expression level | 1.00 | 1.33 |
| SD | 0.06 | 0.08 |
| p | | p < 0.01 |

(Comparison with the unadded group by paired T-test (two-tailed), expressed as a ratio to the unadded group set at 1)

**[Table 2]**

| Table 2: Promotion of GBA expression due to cis-3-hexenol addition | | |
|---|---|---|
| | cis-3-Hexenol unadded | cis-3-Hexenol 0.5 mM |
| Gene expression level | 1.00 | 1.08 |
| SD | 0.02 | 0.04 |
| p | | p < 0.05 |

(Comparison with the unadded group by paired T-test (two-tailed), expressed as a ratio to the unadded group set at 1)

**[Table 3]**

| Table 3: Inhibition of COX2 expression due to cis-3-hexenol addition | | | |
|---|---|---|---|
| Substance name | cis-3-Hexenol unadded | cis-3-Hexenol 0.5 mM | cis-3-Hexenol 1 mM |
| Gene expression level | 1.00 | 0.89 | 0.84 |
| SD | 0.05 | 0.05 | 0.04 |
| p | | p < 0.1 | p < 0.05 |

(Comparison with the unadded group by Dunnett's test, expressed as a ratio to the unadded group set at 1)

**[Table 4]**

| Table 4: Inhibition of MIF expression due to cis-3-hexenol addition | | | |
|---|---|---|---|
| Substance name | cis-3-Hexenol unadded | cis-3-Hexenol 0.5 mM | cis-3-Hexenol 1 mM |
| Gene expression level | 1.00 | 0.95 | 0.93 |
| SD | 0.01 | 0.03 | 0.02 |
| p | | < 0.1 | < 0.05 |

(Comparison with the unadded group by Dunnett's test, expressed as a ratio to the unadded group set at 1)

As shown in Tables 1 to 4, when cis-3-hexenol was added, FLG and GBA expressions were significantly increased, and expressions of COX2 and MIF were significantly decreased. From the above results, it was suggested that promoting FLG and GBA expressions and inhibiting expressions of COX2 and MIF can contribute to moisturization, moisturization, skin barrier function improvement, anti-inflammation, hyaluronic acid increase, and/or melanin production inhibition.

### Example 2: Effect of cis-3-hexenol on fibroblast

Regarding cis-3-hexenol that was found to have an effect in keratinocytes in Example 1, in the present Example, human neonatal dermal fibroblast cell line NB1RGB cells (RIKEN BRC, Japan) were used in place of keratinocytes, and HAS2 gene expression level was measured with the same materials and methods as in Example 1, except that a primer (Hs00193435_m1) for HAS2 (human hyaluronan synthase 2) encoding a hyaluronic acid-synthesizing enzyme was used.

Results are shown in Table 5 below.

**[Table 5]**

| Table 5: Promotion of HAS2 expression due to cis-3-hexenol addition | | |
|---|---|---|
| Substance name | cis-3-Hexenol unadded | cis-3-Hexenol 0.05 mM |
| Gene expression level | 1.00 | 3.16 |
| SD | 0.28 | 0.38 |
| p | | p < 0.05 |

(Compared with the unadded group by paired T-test (two-tailed); expressed as a ratio to the unadded group set at 1)

According to the results of Table 5, cis-3-hexenol significantly increased expression of HAS2 in dermal fibroblasts. From these results, it was suggested that synthesis of dermal hyaluronic acid can be promoted by HAS2 expression promotion due to cis-3-hexenol, thereby contributing to moisturization.

### Example 3: Effect of cis-3-hexenol on hyaluronic acid level

In the present Example, it was confirmed whether or not cis-3-hexenol, which was found to have an HAS2 gene expression promoting effect in dermal fibroblasts in Example 2, also has an effect of increasing hyaluronic acid levels in dermal fibroblasts.

### 3-1: Cell culture

The same NB1RGB cells as in Example 2 were seeded in Eagle's Minimal Essential Medium (EMEM, Cat No. 051-07615, Wako, Japan) comprising 10.0% (v/v) Fetal Bovine Serum (FBS, Cat No. SH30071.03, Hyclone, UK) and 1.0% (v/v) antifungal agent (Antibiotic-Antimycotic 100X, Cat No. 15240-062, Invitrogen, USA) in a 24-well plate (Cat No. 3526, Corning, USA) at a density of 10.0 × 10⁴ cells/well and cultured within a CO2 incubator (CO2 concentration of 5%, 37°C) for 24 h. After 24 h, the medium was removed and replaced with a medium to which cis-3-hexenol (CAS: 928-96-1, Wako Pure Chemical Industries, Ltd.) so as to have the final concentration shown in Table 6 below, and the cells were further cultured within the CO2 incubator for 48 h. As the control, a medium not comprising cis-3-hexenol was used.

### 3-2: Cell-activating effect evaluation

According to the following method, the effect of cis-3-hexenol on proliferation of dermal fibroblasts was evaluated.

A 96-well plate from which the medium was removed was washed with 100 µL of PBS(-). 100 µL of 0.5 mg/mL 3-(4,5-dimethylthiazol-2-yl)-2,5-diphenyltetrazolium bromide (MTT, CAS No. 298-93-1, Sigma-Aldrich, USA) solution was then added and incubated within a CO2 incubator for 2 h. After removing the MTT solution and washing with 100 µL of PBS(-), 200 µL of 2-propanol (CAS No. 67-63-0, Wako, Japan) was added to dissolve the insoluble formazan. A dye was uniformly dispersed within the 96-well plate, and then absorbance (OD570) at 570 nm was measured using a microplate reader (SPARK^{®}, 10M TECAN, Switzerland). Setting OD570 of the control group at 100%, OD570 of the cis-3-hexenol-added group, i.e., the effect on cell proliferation, was calculated as the cell-activating effect (%) of cis-3-hexenol. In the analysis of cell-activating effect, the average value of 96-well plate × 3 wells was used for each treatment group.

### 3-3: Measurement of hyaluronic acid

A supernatant of the culture cultured in 3-1 was dispensed into a new 96-well plate and frozen for storage (-80°C). The hyaluronic acid level in the culture supernatant was measured by sandwich ELISA, and the hyaluronic acid production promoting effect of cis-3-hexenol was evaluated.

100 µL of Hyaluronan Binding Protein (HABP, Cat No. BC40, Hokudo, Japan, 1:5500) solution prepared in PBS was added to a high-binding 96-well plate and incubated overnight at 4°C. After removing the immobilized HABP solution and washing with 200 µL of PBS-T solution, 150 µL of 1% BSA solution was added and incubated at room temperature for 1 h. After removing the BSA solution and washing with 200 µL of PBS-T, 100 µL of culture supernatant diluted 100-fold with PBS(-) was added and incubated at room temperature for 1 h. As the standard substance, sodium hyaluronate (Cat No. 087-04511, Wako, Japan) was used. After removing the culture supernatant and washing with 200 µL of PBS-T, 100 µL of biotin-labeled HABP (Cat No. BC41, Hokudo, Japan, 1:2000) solution prepared in 0.5% BSA-containing PBS(-) was added and left to stand overnight at 4°C. After removing the biotin-labeled HABP solution and washing with 200 µL of PBS-T, 100 µL of Streptavidin-HRP solution (1:10000) prepared in 0.5% BSA-containing PBS(-) was added and left to stand at room temperature for 30 min. After removing the Streptavidin-HRP solution and washing with 200 µL of PBS-T, 100 µL of ABTS solution was added, and color development was confirmed. The dye was uniformized within the 96-well plate, and then absorbance (OD405) at 405 nm was measured using a microplate reader.

Setting OD405 of the control group at 100%, the hyaluronic acid production rate of cis-3-hexenol was calculated. In addition, the OD405 of the control and the cis-3-hexenol-added sample were each divided by OD570 measured in 3-2 to calculate the hyaluronic acid production rate per cell. Setting the hyaluronic acid production rate per cell of the control group at 100%, the hyaluronic acid production rate per cell due to cis-3-hexenol was calculated. In the analysis of hyaluronic acid production rate, the average value of 96-well plate × 3 wells was used for each treatment group.

The results of 3-2 confirmed that no significant increase or significant decrease in proliferation of fibroblasts was observed, and cis-3-hexenol had no cell-activating effect or toxicity. The results of 3-3 are shown in Table 6 as the hyaluronic acid production rate due to cis-3-hexenol when hyaluronic acid production rate of the control was set at 100%.

**[Table 6]**

| Table 6: Hyaluronic acid production rate (%) per cell due to cis-3-hexenol addition | | |
|---|---|---|
| Substance name | cis-3-Hexenol unadded | cis-3-Hexenol 50 µM |
| Gene expression level | 100.0 | 121.5 |
| SD | 9.2 | 2.3 |
| p | | p < 0.05 |

(Compared with the unadded group by unpaired T-test (two-tailed); expressed as a ratio to the unadded group set at 100)

Due to the addition of cis-3-hexenol, the hyaluronic acid production rate per cell was significantly increased. From the above results, it was confirmed that cis-3-hexenol contributes to a hyaluronic acid increase in fibroblasts. In addition, when considering the results of Example 2, it was suggested that the hyaluronic acid increasing effect of cis-3-hexenol is possibly due to the promotion of HAS2 expression.

According to the above results, cis-3-hexenol was found to promote FLG, GBA, and HAS2 gene expressions, inhibit COX2 and MIF gene expressions, and increase hyaluronic acid, and was further expected to exert preferable effects on the skin, such as moisturization, skin barrier function improvement, anti-inflammation, and melanin production inhibition.

## Claims

1. A moisturizer containing cis-3-hexenol as an active component.

2. A skin barrier function improving agent containing cis-3-hexenol as an active component.

3. An anti-inflammatory agent containing cis-3-hexenol as an active component.

4. A hyaluronic acid increasing agent containing cis-3-hexenol as an active component.

5. An FLG gene expression promoter containing cis-3-hexenol as an active component.

6. A GBA gene expression promoter containing cis-3-hexenol as an active component.

7. A COX2 gene expression inhibitor containing cis-3-hexenol as an active component.

8. An HAS2 gene expression promoter containing cis-3-hexenol as an active component.
